# EUROPÄISCHE PATENTSCHRIFT

(11) **EP 0 511 164 B1**
(45) Veröffentlichungstag und Bekanntmachung des Hinweises auf die Patenterteilung: **08.05.1996**
(21) Anmeldenummer: 92810284.7
(22) Anmeldetag: 16.04.1992
(51) Int. Cl.: C07D 495/06, H01B 1/12

(54) **Verfahren zur Herstellung von 5,6,11,12-Tetrathiotetracen**
Process for the preparation of 5,6,11,12-tetrathiotetracene
Procédé de préparation de 5,6,11,12-tétrathiotétracène

(30) Priorität: 25.04.1991 CH 1236/91
(43) Veröffentlichungstag der Anmeldung: 28.10.1992
(73) Patentinhaber: CIBA-GEIGY AG, CH-4002 Basel (CH)
(72) Erfinder: Alder, Alex, Dr., CH-4422 Arisdorf (CH)

(56) Entgegenhaltungen:
- CHEMICAL ABSTRACTS, vol. 112, no. 19, 7. Mai 1990, Columbus, Ohio, US; abstract no. 178743P, R. MEDNE ET AL.: 'New methods for synthesis of tetrathiotetracene' Seite 746 ; & Latv. PSR Zinat. Akad. Vestis, Kom. Ser. 1989, (5), 633-634
- JOURNAL OF THE AMERICAN CHEMICAL SOCIETY. Bd. 100, Nr. 14, 1978, GASTON, PA US Seiten 4612 - 4614; F. B. BRAMWELL ET AL.: 'Electron spin resonance studies of sulfur-based donor heterocycles: 33S couplings'
- CHEMICAL ABSTRACTS, vol. 92, no. 25, 23. Juni 1980, Columbus, Ohio, US; abstract no. 215353C, K. BALODIS ET AL.: 'Synthesis and some reactions of 5,6,11,12-tetrachlorotetracene' Seite 618; & Sint. Issled. Biol. Soedin., Tezisy Dokl. Konf. Molodykh Uch, 6th 1978, 26-7

## Beschreibung

Die vorliegende Erfindung betrifft ein Verfahren zur Herstellung von 5,6,11,12-Tetrathiotetracen durch Umsetzung von 5,6,11,12-Tetrachlortetracen mit einem Gemisch aus Thioharnstoff und Schwefel in einem polaren aprotischen Lösungsmittel.

R. Medne et al. beschreiben im Latv. PSR Zinat. Akad. Vestis, Khim. Ser., S. 633 (1989) die Umsetzung von 5,6,11,12-Tetrachlortetracen mit Thioharnstoff in Hexamethylphosphorsäuretriamid unter Inertgasatmosphäre. Hierbei wird 5,6,11,12-Tetrathiotetracen in mässigen Ausbeuten und in ungenügender Reinheit gebildet.

Es wurde nun gefunden, dass man sowohl die Ausbeute als auch die Reinheit des Produkts erheblich steigern und sogar auf eine Schutzgasatmosphäre verzichten kann, wenn man bei dieser Umsetzung zusätzlich Schwefel mitverwendet.

Gegenstand der Erfindung ist somit ein Verfahren zur Herstellung von 5,6,11,12-Tetrathiotetracen der Formel I 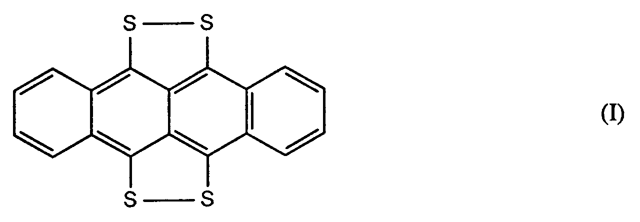
durch Umsetzung von 5,6,11,12-Tetrachlortetracen der Formel II 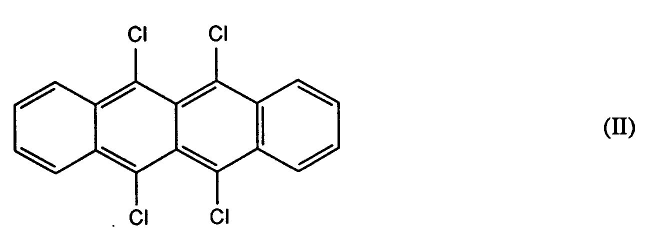
mit Thioharnstoff in Gegenwart eines polaren aprotischen Lösungsmittels, das dadurch gekennzeichnet ist, dass man die Umsetzung mit einem Gemisch aus Thioharnstoff und Schwefel durchführt.

Geeignete Lösungsmittel sind zum Beispiel N-alkylierte Säureamide und Lactame (zum Beispiel Dimethylformamid, Diethylformamid, Dimethylacetamid, Tetramethylharnstoff, Hexamethylphosphorsäuretriamid, N-Methylpyrrolidon), Ether (zum Beispiel Dipropylether, Dibutylether, Tetrahydrofuran, Dioxan, Ethylenglykoldimethyl- oder -diethylether, Diethylenglykoldimethyl- oder -diethylether), Ester und Lactone (zum Beispiel Essigsäureethyl-, -propyl- oder -butylester, Propionsäureethylester, Buttersäureethylester, Butyrolacton, Valerolacton), und Sulfone und Sulfoxide (zum Beispiel Dimethylsulfoxid, Dimethylsulfon, Tetramethylensulfon). Bevorzugte Lösungsmittel sind N-alkylierte Carbonsäureamide und Lactame; besonders bevorzugt ist Dimethylacetamid.

Die Reaktionstemperatur kann zum Beispiel 70 bis 250 °C, bevorzugt 80 bis 200 °C und insbesondere bevorzugt 80 bis 160 °C betragen. In einer bevorzugten Ausführungsform wird die Reaktion zunächst bis zu 20 Stunden, bevorzugt bis zu 10 Stunden bei einer Temperatur von 70 bis 120 °C, bevorzugt 80 bis 110 °C, und anschliessend bis zu 6 Stunden, bevorzugt bis zu 4 Stunden bei einer Temperatur von mehr als 120 °C bis zu 250 °C, bevorzugt 130 °C und bis zu 200 °C, insbesondere bevorzugt 130 °C und bis zu 160 °C durchgeführt

Der Thioharnstoff und der Schwefel können in äquivalenten Mengen eingesetzt werden, bezogen auf das 5,6,11,12-Tetrachlortetracen. Bevorzugt wird ein Überschuss an Thioharnstoff verwendet. In einer bevorzugten Ausführungsform verwendet man 4,2 bis 42 Moläquivalente, bevorzugt 10 bis 15 Moläquivalente Thioharnstoff, und 2,5 bis 10 Moläquivalente, bevorzugt 4 bis 6 Moläquivalente Schwefel, bezogen auf 1 Mol 5,6,11,12-Tetrachlortetracen.

Die Konzentration an 5,6,11,12-Tetrachlortetracen im Reaktionsgemisch beträgt zweckmässig von 0,01 bis 0,20, bevorzugt 0,06 bis 0,12 Mol pro Liter Lösungsmittel.

Die Reaktion kann unter erhöhtem Druck durchgeführt werden; zweckmässig ist die Durchführung unter Normaldruck. Es ist ferner möglich, die Reaktion unter Schutzgasatmosphäre durchzuführen. Ein besonderer Vorteil des erfindungsgemässen Verfahrens besteht darin, dass keine Schutzgasatmosphäre notwendig und die Durchführung an Luft möglich ist.

Das erfingungsgemässe Verfahren kann zum Beispiel so durchgeführt werden, dass man den Schwefel im Lösungsmittel vorlegt und erwärmt. Zu der erwärmten Lösung gibt man den Thioharnstoff und das 5,6,11,12-Tetrachlortetracen und rührt einige Zeit bei dieser Temperatur. Danach erhöht man die Temperatur und rührt noch einige Zeit weiter.

Die Isolierung des 5,6,11,12-Tetrathiotetracens erfolgt in an sich bekannter Weise zum Beispiel durch Hydrolyse des Reaktionsgemisches mit verdünnten Mineralsäuren, wobei das Produkt ausfällt und abfiltriert werden kann. Das Produkt kann danach zum Beispiel durch Auswaschen mit Nichtlösungsmitteln, durch Umkristallisation oder durch Sublimation weiter gereinigt werden, wobei durch blosses Auswaschen bereits eine hohe Reinheit erzielt werden kann.

5,6,11,12-Tetrathioteracen ist ein bekannter Elektronendonator, der mit Elektronenakzeptoren wie zum Beispiel Halogenen elektrisch leitende Radikalkationensalze (organische Metalle) bildet, die zur antistatischen Ausrüstung von Kunststoffen oder als Elektroden verwendet werden können.

Das nachfolgende. Beispiel erläutert die Erfindung.

Beispiel: In einem offenen Gefäss werden 6 g Schwefel und 225 ml Dimethylacetamid auf 90 °C erwärmt. Zu dieser Lösung werden unter Rühren 30 g Thioharnstoff und danach 13,5 g 5,6,11,12-Tetrachlortetracen gegeben. Dann wird das Reaktionsgemisch zunächst 6 Stunden bei 90 °C und dann 2 Stunden bei 140 °C gerührt. Die schwarzgrüne Lösung wird auf 100 °C abgekühlt und 1125 ml Wasser, das 4,5 ml HCl (5N) enthält, innerhalb von 10 Minuten zugetropft. Der gebildete Niederschlag wird bei 40 °C abfiltriert und mit 11 Wasser, 1 l Ethanol, 1 l Benzol und 200 ml Diethylether gewaschen. Danach trocknet man das Produkt bei Raumtemperatur in Vakuum. Man erhält 11,3 g (85 % der Theorie) 5,6,11,12-Tetrathiotetracen mit einer Reinheit von mehr als 98 %.

## Patentansprüche

1. Verfahren zur Herstellung von 5,6,11,12-Tetrathiotetracen der Formel I 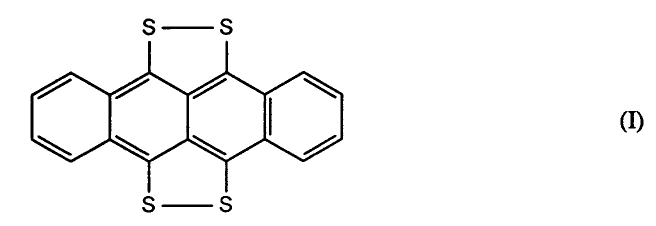 durch Umsetzung von 5,6,11,12-Tetrachlortetracen der Formel II 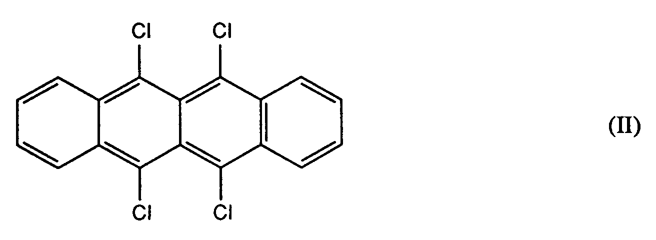 mit Thioharnstoff in Gegenwart eines polaren aprotischen Lösungsmittels, dadurch gekennzeichnet, dass man die Umsetzung mit einem Gemisch aus Thioharnstoff und Schwefel durchführt.

2. Verfahren gemäss Anspruch 1, dadurch gekennzeichnet, dass es sich bei dem Lösungsmittel um N-alkylierte Carbonsäureamide und Lactame handelt.

3. Verfahren gemäss Anspruch 2, dadurch gekennzeichnet, dass es sich bei dem Lösungsmittel um Dimethylacetamid handelt.

4. Verfahren gemäss Anspruch 1, dadurch gekennzeichnet, dass die Reaktionstemperatur von 70 bis 250 °C beträgt.

5. Verfahren gemäss Anspruch 1, dadurch gekennzeichnet, dass die Reaktionstemperatur von 80 bis 200 °C beträgt.

6. Verfahren gemäss Anspruch 1, dadurch gekennzeichnet, dass die Reaktion zunächst für bis zu 20 Stunden bei einer Temperatur von 70 bis 120 °C und danach für bis zu 6 Stunden bei einer Temperatur von mehr als 120 °C bis zu 250 °C durchgeführt wird.

7. Verfahren gemäss Anspruch 6, dadurch gekennzeichnet, dass die Reaktion zunächst für bis zu 20 Stunden bei einer Temperatur von 80 bis 110 °C und danach für bis zu 6 Stunden bei einer Temperatur von mehr als 130 °C bis zu 200 °C durchgeführt wird.

8. Verfahren gemäss Anspruch 1, dadurch gekennzeichnet, dass 4,2 bis 42 Moläquivalente Thioharnstoff und 2,5 bis 10 Moläquivalente Schwefel pro Mol 5,6,11,12-Tetrachlortetracen verwendet werden.

9. Verfahren gemäss Anspruch 8, dadurch gekennzeichnet, dass 10 bis 15 Moläquivalente Thioharnstoff und 4 bis 6 Moläquivalente Schwefel pro Mol 5,6,11,12-Tetrachlortetracen verwendet werden.

10. Verfahren gemäss Anspruch 8, dadurch gekennzeichnet, dass die Menge an 5,6,11,12-Tetrachlortetracen 0,01 bis 0,20 Mol pro Liter Lösungsmittel beträgt.

11. Verfahren gemäss Anspruch 1, dadurch gekennzeichnet, dass es ohne Schutzgasatmosphäre durchgeführt wird.

## Claims

1. A process for the preparation of 5,6,11,12-tetrathiotetracene of formula I 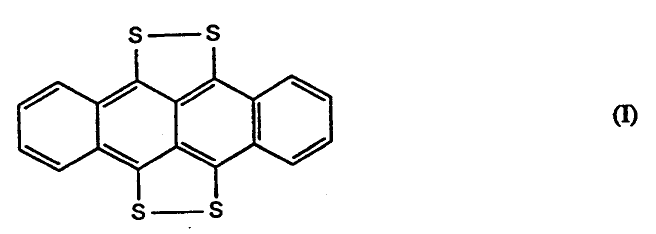 by reacting 5,6,11,12-tetrachlorotetracene of formula II 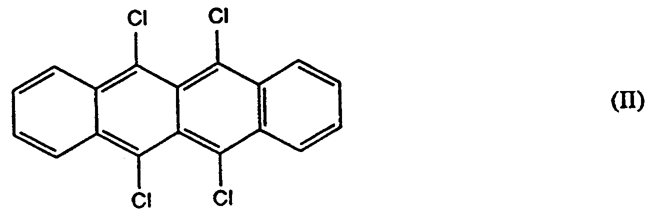 with thiourea in the presence of a polar aprotic solvent, which comprises carrying out the reaction with a mixture of thiourea and sulfur.

2. A process according to claim 1, wherein the solvent is an N-alkylated carboxamide or a lactam.

3. A process according to claim 2, wherein the solvent is dimethylacetamide.

4. A process according to claim 1, wherein the reaction temperature is from 70 to 250°C.

5. A process according to claim 1, wherein the reaction temperature is from 80 to 200°C.

6. A process according to claim 1, wherein the reaction is carried out initially for up to 20 hours at a temperature from 70 to 120°C, and thereafter for up to 6 hours at a temperature of from more than 120°C up to 250°C.

7. A process according to claim 6, wherein the reaction is carried out initially for up to 20 hours at a temperature from 80 to 110°C, and thereafter for up to 6 hours at a temperature of from more than 130°C up to 200°C.

8. A process according to claim 1, which comprises using 4.2 to 42 molar equivalents of thiourea and 2.5 to 10 molar equivalents of sulfur per mole of 5,6,11,12-tetrachlorotetracene.

9. A process according to claim 8, which comprises using 10 to 15 molar equivalents of thiourea and 4 to 6 molar equivalents of sulfur per mole of 5,6,11,12-tetrachlorotetracene.

10. A process according to claim 8, wherein the quantity of 5,6,11,12-tetrachlorotetracene is from 0.01 to 0.20 mol per litre of solvent.

11. A process according to claim 1, which is carried out in the absence of an inert gas atmosphere.

## Revendications

1. Procédé pour préparer du 5,6,11,12-tétrathiotétracène de formule I 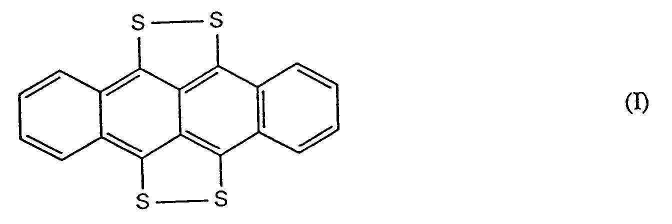 par réaction de 5,6,11,12-tétrachlorotétracène de formule II 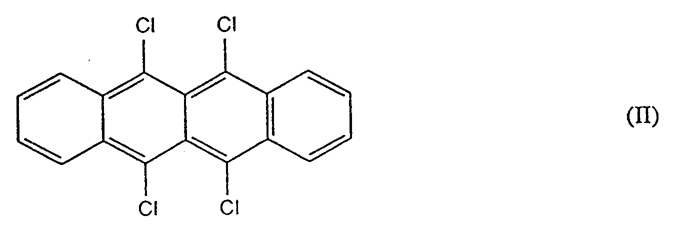 avec la thiourée en présence d'un solvant aprotique polaire, caractérisé en ce qu'on met en oeuvre la réaction avec un mélange de thiourée et de soufre.

2. Procédé selon la revendication 1, caractérisé en ce que, pour ce qui est du solvant, il s'agit de carboxamides et de lactames N-alkylés.

3. Procédé selon la revendication 2, caractérisé en ce que, pour ce qui est du solvant, il s'agit du diméthylacétamide.

4. Procédé selon la revendication 1, caractérisé en ce que la température de la réaction est de 70 à 250°C.

5. Procédé selon la revendication 1, caractérisé en ce que la température de la réaction est de 80 à 200°C.

6. Procédé selon la revendication 1, caractérisé en ce que la réaction est mise en oeuvre d'abord pendant un laps de temps allant jusqu'à 20 heures à une température de 70 à 120°C, puis pendant un laps de temps allant jusqu'à 6 heures à une température supérieure à 120°C et allant jusqu'à 250°C.

7. Procédé selon la revendication 6, caractérisé en ce que la réaction est mise en oeuvre d'abord pendant un laps de temps allant jusqu'à 20 heures à une température de 80 à 110°C, puis pendant un laps de temps allant jusqu'à 6 heures à une température supérieure à 130°C et allant jusqu'à 200°C.

8. Procédé selon la revendication 1, caractérisé en ce qu'on utilise de 4,2 à 42 équivalents molaires de thiourée et de 2,5 à 10 équivalents molaires de soufre par mole de 5,6,11,12-tétrachlorotétracène.

9. Procédé selon la revendication 8, caractérisé en ce qu'on utilise de 10 à 15 équivalents molaires de thiourée et de 4 à 6 équivalents molaires de soufre par mole de 5,6,11,12-tétrachlorotétracène.

10. Procédé selon la revendication 8, caractérisé en ce que la quantité de 5,6,11,12-tétrachlorotétracène est de 0,01 à 0,20 mole par litre de solvant.

11. Procédé selon la revendication 1, caractérisé en ce qu'il est mis en oeuvre sans atmosphère d'un gaz inerte.
